(19) Europäisches Patentamt
European Patent Office
Office européen des brevets

(11) Publication number: 0 327 214 A2

(12) **EUROPEAN PATENT APPLICATION**

(21) Application number: 89300387.1

(22) Date of filing: 17.01.89

(51) Int. Cl.4: A01N 43/80 , A01N 65/00 , A01N 61/00 , C11B 9/00 , A61L 9/04

(30) Priority: 01.02.88 JP 21701/88

(43) Date of publication of application: 09.08.89 Bulletin 89/32

(84) Designated Contracting States: DE GB

(71) Applicant: Kurita Water Industries Ltd.
4-7, Nishi-Shinjuku 3-chome
Shinjuku-ku Tokyo-to(JP)

(72) Inventor: Sekikawa, Ayako c/o Kurita Water Industries Ltd.
4-7, Nishishinjuku 3-Chome
Shinjuku-Ku Tokyo(JP)
Inventor: Sugi, Hideo c/o Kurita Water Industries Ltd.
4-7, Nishishinjuku 3-Chome
Shinjuku-Ku Tokyo(JP)
Inventor: Takahashi, Ryoichi Kurita Water Industries Ltd.
4-7, Nishishinjuku 3-Chome
Shinjuku-Ku Tokyo(JP)

(74) Representative: Jenkins, Peter David et al
PAGE, WHITE & FARRER 54 Doughty Street
London WC1N 2LS(GB)

# (54) Clathrate compound and a method of producing the same.

(57) A clathrate compound formed by a hydroquinone compound represented by general formula (I)

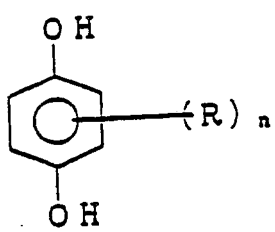

(I)

where R is a branched alkyl group having 3 to 7 carbon atoms, and n is 1 or 2; and a water-soluble microbicide or natural essential oil. A hydroquinone compound is mixed with a water-soluble microbicide or essential oil, or a solution or dispersion of the hydroquinone compound is mixed with a solution containing the water-soluble microbicide or with the essential oil, whereby a solid reaction product is obtained. It is separated from a liquid phase to yield a desired clathrate compound.

EP 0 327 214 A2

## Clathrate Compound and a Method of Producing the Same

This invention relates to a clathrate compound and a method of producing it. More particularly, it relates to a novel clathrate compound which is useful as a sustained release antimicrobial agent, insecticide or aromatic, and a method of producing it.

The cooling water systems in various industrial facilities, water systems in paper and pulp making plants, etc. suffer from various kinds of trouble that are caused by the deposition therein of a slime of animal, vegetable or microbial origin.

In order to prevent the deposition of any such slime and thereby the occurrence of any such trouble, it has been customary to use an antimicrobial agent (or slime controll agent), as it is easy to use and is also inexpensive. While there are known various kinds of antimicrobial agents, 5-chloro-2-methyl-4-isothiazoline-3-one (hereinafter referred to simply as "CMI"), which is represented by formula (II) below, is particularly widely used as a slime controll agent, bactericide, algicide or fungicide in cooling water systems, water systems in the paper and pulp industry, swimming pools and other water systems, or as a preservative for polymer emulsions, adhesives and paints since it has a high degree of antimicrobial activity:

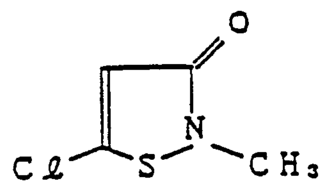

(II)

CMI is usually produced by:

(1) the halogenation of -thioketoamide in an inert organic ester solvent, such as an acetic acid ester; or

(2) the treatment of a -substituted thiocyanoacrylamide or thiosulfatoacrylamide with an acid to obtain isothiazolone and the halogenation thereof, as disclosed in Japanese Patent Publication No. 21240/1971.

Neither of these two methods (1) and (2) can, however, make a product which is composed solely of CMI. They can make only a product which contains as a byproduct 2-methyl-4-isothiazoline-3-one (hereinafter referred to simply as "MI") which is represented by formula (III) and has only an antimicrobial activity which is as low as one-tenth of that of CMI:

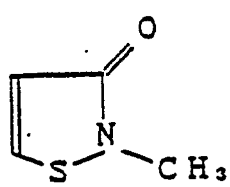

(III)

Moreover, there is not known any method that can separate only CMI from the reaction product. There is no alternative but to use a mixture containing MI having a low degree of antimicrobial activity.

Although CMI has a high degree of antimicrobial activity, its use requires a great deal of care, as it is highly irritant to the skin. When it is used in water, it can hardly maintain its antimicrobial activity for a long period of time, as it easily reacts with organic substances in water, such as amines and reducing substances, and thereby loses its activity. No antifouling paint containing CMI can maintain its antifouling effect for a long period of time when it is used in water, as CMI is easily soluble in water.

The known water-soluble antimicrobial agents have a number of serious drawbacks including the toxicity which compels a great deal of care to be taken when they are used, a rapid reduction in antimicrobial activity, and high solubility in water, as hereinabove pointed out.

The same is true with the known insecticides and aromatics. There has been a strong desire for a sustained release insecticide or aromatic which can maintain its insecticidal or aromatic effect for a long period of time and yet can be used easily and safely.

Objects and Summary of the Invention:

The present invention aims to provide a clathrate compound which can form an improved sustained release antimicrobial agent, insecticide or aromatic overcoming the drawbacks of the prior art as hereinabove pointed out, and a method of producing it.

The present invention also aims to provide a clathrate compound which can be used effectively for the formation into a powder, stabilization, concentration, separation or purification of a water-soluble microbicide or natural essential oil, and a method of producing it.

The clathrate compound of this invention comprises a hydroquinone compound represented by general formula (I) below; and a water-soluble microbicide or natural essential oil:

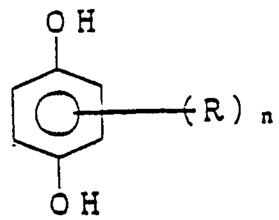

(I)

where R is a branched alkyl group having 3 to 7 carbon atoms, and n is 1 or 2.

The method of this invention comprises mixing a hydroquinone compound of formula (I) with a water-soluble microbicide or natural essential oil, or mixing a solution or dispersion of a hydroquinone compound of formula (I) in a solution containing a water-soluble microbicide, or natural essential oil, whereby a solid reaction product is obtained; and separating the solid product.

According to this invention, therefore, a water-soluble microbicide or natural essential oil is used as the guest compound, and a hydroquinone compound as the host compound in which the guest compound is included to form a clathrate by the host compound.

Preferred embodiments of the present invention will now be described in greater detail.

Referring in detail to this invention, it is possible to use a hydroquinone compound of formula (I) in which R is, for example, a t-butyl, 1,1-dimethylpropyl, 1,1,2,2-tetramethylpropyl, isopropyl or isobutyl group. More specifically, it is possible to use, for example, 2,5-di-tert-butylhydroquinone, which is represented by formula (IV):

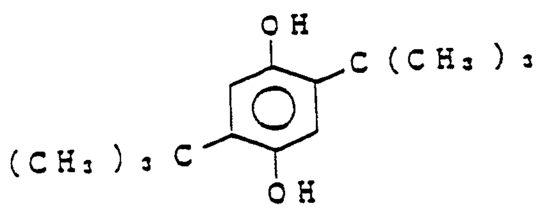

(IV)

When a water-soluble microbicide is employed, it is possible to use any that can form a clathrate compound with a hydroquinone compound. More specifically, it is possible to use CMI or hydrazine. They are both widely used as an effective microbicide. It is, however, possible to use any other appropriate microbicide, if it can form a clathrate with formula (I).

Specific examples of the natural essential oils which can be employed for the purpose of this invention are cineole, hinoki oil, fragrant olive oil, jasmine oil, lemon oil, quassia oil, cinnamon oil, menthol, rosemary oil, palmarosa oil, lavender oil, spearmint oil and It is, of course, possible to use any other appropriate essential oil, if it can form a clathrate with formula (I).

The clathrate compound of this invention, which is formed by a hydroquinone compound as the host compound and a water-soluble microbicide or natural essential oil as the guest compound, can be produced easily by the method of this invention, whether a solvent may be used or not.

When a solvent is used, it is possible to use any ordinary water-soluble solvent such as methanol, ethanol or acetone, or any non water-soluble solvent or dispersant such as benzene, toluene, xylene or chloroform. A solution or dispersion of a hydroquinone compound is mixed with the guest compound or a mixture thereof with impurities, whereby the host and guest compounds are caused to react with each other. A clathrate compound is obtained as a solid precipitate and is separated by a customary method of

filtration.

Although the clathrate compound of this invention can be produced easily by employing a solvent as hereinabove described, it is more advantageous to carry out this invention without using any solvent. The use of a solvent gives rise to various problems or inconveniences including the following:

(1) It is necessary to choose an appropriate solvent;

(2) It is relatively difficult to select the appropriate conditions for the reaction;

(3) It is necessary to employ facilities for protecting the workers and their working environment, particularly when an organic solvent is used;

(4) The waste resulting from the separation of the solid from the liquid requires special disposal; and

(5) The host compound can be recovered in the clathrate product only at a relatively low rate.

All of these problems can be overcome if no solvent is used.

When no solvent is used, a clathrate compound containing a water-soluble microbicide as the guest compound can be produced if a hydroquinone compound is directly added into an aqueous solution of the microbicide and the mixture is stirred. If the guest compound is natural essential oil, the hydroquinone compound is directly admixed in the essential oil, as the latter is a liquid.

The aqueous solution of the microbicide does not necessarily need to be one containing only the microbicide, but may further contain any byproduct or other impurities. When essential oil is used, it may also contain impurities. According to this invention, the hydroquinone compound reacts with only the microbicide or essential oil and thereby forms a clathrate compound in which only the microbicide or essential oil is included in the host compound, even if the microbicide solution or essential oil which is employed may contain impurities.

Although it is possible to employ a reaction temperature in the range of 0°C to 100°C, a preferred range is from about 10°C to about 30°C. It is sufficient to continue the reaction for a period of, say, 0.5 to 24 hours.

The clathrate compound which is obtained as a result of the reaction is usually in solid form. It is separated from the liquid layer, washed with water, and dried.

The NMR analysis of the clathrate compound which has been obtained can be used to ascertain that only the intended guest compound is included in the host compound. The NMR analysis can also be used to determine the molar ratio of the guest compound in the clathrate compound.

The clathrate compound of this invention is usually a powdery solid and is, therefore, easy to form into tablets or any other shape. It is low in toxicity and is, therefore, easy to use, insofar as the water-soluble microbicide or essential oil is imprisoned in the host compound. Moreover, it is unlikely to react with any other substance and lower its own antimicrobial or insecticidal activity while it is in use.

The clathrate compound of this invention containing a water-soluble microbicide is useful as a sustained release antimicrobial agent which can maintain its antimicrobial activity for a very long time when it is used in water, since the microbicide is dissolved in water only slowly.

When the clathrate compound of this invention is used as a sustained release antimicrobial agent, it can be employed in a variety of ways as will be described below:

(1) The water to be treated is passed through a column packed with the agent;

(2) A bag or cartridge of a material which is permeable to water, but not soluble therein, is filled with the agent and is submerged or floated in the water to be treated;

(3) The agent in its powdery form or any product molded therefrom is dispersed in the water to be treated;

(4) A mixture of the agent with a paint or any other resinous coating material is applied to the surfaces of equipment in the water system to be protected;

(5) A mixture of the agent with ink (or medium alone) is applied by printing onto the surfaces to be protected; and

(6) The agent is caused by any other appropriate method to adhere to the surfaces to be protected.

The clathrate compound of this invention containing an aromatic essential oil provides a sustained release aromatic which releases aroma in a sustained way which can be varied easily if the particle size of the powder of the clathrate compound or the shape of any molded product thereof is so altered as to have a differently sized area of contact with the air.

When the clathrate compound of this invention is used as a sustained release aromatic, it can be employed in a variety of ways as will be described below:

(1) The aromatic in powder form is placed in a container having an opening or openings;

(2) The aromatic is molded into an appropriate shape;

(3) A mixture of the aromatic with a paint or any other resinous coating material is applied to an appropriate object;

(4) A mixture of the aromatic with ink (or medium alone) is applied by printing onto the surface of an appropriate object; and

(5) The aromatic is caused by any other appropriate method to adhere to the surface of the object.

The clathrate compound of this invention is also useful for the formation into a powder, stabilization or concentration of a water-soluble microbicide or essential oil. It can also be used for the separation or purification of a specific water-soluble microbicide or essential oil, insofar as it is the product of a highly selective reaction between the specific compounds. For example, while it has hitherto been difficult to separate only CMI from a mixture of CMI and MI, it is possible to obtain CMI of high purity by separating CMI from the clathrate compound of this invention, since the hydroquinone compound, which is employed as the host compound for forming the clathrate compound of this invention, reacts with only CMI, even if a mixture of CMI and MI may be employed. The water-soluble microbicide, such as CMI, can be separated from the clathrate compound:

(1) if the clathrate compound is immersed in water; or

(2) if it is dissolved in an organic solvent and water is added into its solution, whereby the host compound is caused to settle.

<In either event, the microbicide is dissolved in water and recovered in the form of its aqueous solution.

While the clathrate compound is useful for the formation into a powder, stabilization, concentration, separation or purification of the water-soluble microbicide or essential oil which is employed as the guest compound, it is particularly useful as a sustained release antimicrobial agent containing the water-soluble microbicide as its effective constituent, for a number of reasons including the following:

(1) It can maintain its antimicrobial activity for a long time, as its effective constituent is dissolved in water only slowly;

(2) As it is a solid, it can be formed into tablets or any other shape and is, therefore, easy to use;

(3) It ensures an improved working environment and improved safety, since the microbicide is imprisoned and is, therefore, less toxic and less irritant to the skin; and

(4) Its effective constituent does not undergo any reaction with other substances resulting in a reduction of its antimicrobial activity.

The water-soluble microbicide is imprisoned in solid form in the hydroquinone compound and is, therefore, dissolved in water only slowly. As it is imprisoned, it is less toxic and less irritant to the skin. Moreover, it does not undergo any reaction with other substances resulting in a reduction of the activity of the antimicrobial agent while it is in use. Therefore, the clathrate compound of this invention can be used effectively as a sustained release antimicrobial agent which can maintain its antimicrobial activity for a very long time.

The clathrate compound of this invention is also useful as a sustained release aromatic containing essential oil as its aromatic constituent, for a number of reasons including the following:

(1) It can maintain its aromatic property for a long time, as its aromatic constituent is volatilized into the air only slowly;

(2) As it is a solid, it is easy to handle and is also easy to form into any desired shape; and

(3) It is easily possible to control the degree of volatilization of its aromatic constituent to obtain any desired sustained release property if, for example, the particle size of the clathrate compound is appropriately altered.

Moreover, it is useful as a sustained release insecti cide containing natural essential oil as its insecticidal constituent.

The essential oil also has a solid form in the clathrate compound which it forms withe the hydroquinone compound. The clathrate compound of this invention, therefore, provides, for example, an aromatic which is easy to handle, while a liquid perfume has the drawback of spilling if its container falls.

The aromatic or insecticide can maintain its aromatic or insecticidal effect for a long time, as its effective constituent is imprisoned and is released from the clathrate compound only slowly at a low rate of volatilization. It is possible to control the rate of volatilization of its aromatic or insecticidal constituent by altering the particle size of the clathrate compound and thereby the surface area in which it contacts the air. Moreover, it can be formed easily into various shapes.

The invention will now be described more specifically with reference to several examples thereof. It is, however, to be understood that the following description is not intended to limit the scope of this invention,

but that variations or modifications may be easily made by anybody of ordinary skill in the art without departing from the scope of this invention which is defined by the appended claims.

Example 1:

A mixture of 0.2 g of 2,5-di-tert-butylhydroquinone and 2.0 g of an aqueous solution of a water-soluble microbicide consisting mainly of CMI, which had a microbicide content of 10.4% by weight, was stirred for undergoing reaction at room temperature for four hours, whereby a solid reaction product was obtained. It was separated by filtration, washed with water, and dried.

The integration of the NMR spectrum of the product confirmed that it was a clathrate compound containing 2,5-di-tert-butylhydroquinone and CMI in a ratio of 1:0.36.

Example 2:

One gram of the clathrate compound which had been obtained in Example 1 was dispersed in one liter of pure water. While its dispersion was stirred at 25° C, samples were taken from the dispersion at intervals of time as shown in Table 1, and the amount of CMI dissolved in each sample was determined. The results are shown in Table 1.

Table 1

| Time elapsed (h) | 0.25 | 0.5 | 1.0 | 2.0 | 4.0 | 7.0 |
|---|---|---|---|---|---|---|
| Amount of CMI (wt. %) | 48.8 | 67.2 | 88.0 | 94.8 | 98.8 | 98.4 |

As is obvious from the results shown in Table 1, the clathrate compound of this invention can be used effectively as a sustained release antimicrobial agent maintaining its antimicrobial activity for a long period of time.

Example 3:

A mixture of 1.294 g of 2,5-di-tert-butylhydroquinone and 2.032 g of cineole was stirred at room temperature. Their reaction proceeded rapidly and yielded a white solid reaction product. The integration of the NMR spectrum of the product confirmed that it was a clathrate compound composed of 2,5-di-tert-butylhydroquinone and cineole in a molar ratio of 1:2.33 and having a cineole content of 61.8% by weight.

Example 4:

A sample weighing 2.95 g was taken from the clathrate compound which had been obtained in Example 3, and was placed in a laboratory dish. It was allowed to stand at 25° C in an open system and its weight reduction was determined at intervals of time as shown in Table 2 as a measure of a weight reduction of cineole. The results are shown in Table 2.

Table 2

| Time elaspsed (day or days) | 1 | 5 | 7 | 12 | 22 | 26 | 33 |
|---|---|---|---|---|---|---|---|
| Cineole reduction (wt. %) | 20.8 | 44.2 | 61.2 | 72.1 | 83.9 | 92.8 | 93.1 |

As is obvious from Table 2, the clathrate compound of Example 3 allowed the diffusion of cineole only in a sustained way.

Example 5:

A solution obtained by dissolving 0.2 g of 2,5-di-tert-butylhydroquinone in 0.2 ml of acetone was mixed with 1.5 g of an aqueous solution of a water-soluble microbicide consisting mainly of CMI which had a microbicide content of 10.4% by weight. The mixture was stirred at room temperature for an hour, whereby a solid reaction product was obtained. It was collected by filtration, washed with water, and dried.

The NMR analysis of the product confirmed that it was a clathrate compound containing 2,5-di-tert-butylhydroquinone and CMI in a ratio of 1:0.4.

## Claims

1. A clathrate compound comprising:
a hydroquinone compound represented by general formula (I)

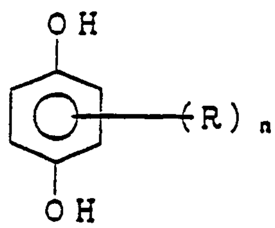

(I)

where R is a branched alkyl group having 3 to 7 carbon atoms, and n is 1 or 2; and
a water-soluble microbicide.

2. A clathrate compound according to claim 1, wherein said hydroquinone compound is 2,5-di-tert-butylhydroquinone.

3. A clathrate compound according to claim 1 or claim 2, wherein said microbicide is 5-chloro-2-methyl-4-isothiazoline-3-one.

4. A clathrate compound comprising:
a hydroquinone compound represented by general formula (I).

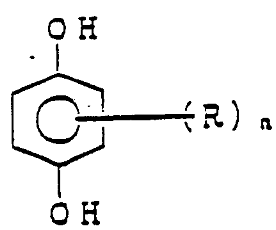

(I)

where R is a branched alkyl group having 3 to 7 carbon atoms, and n is 1 or 2; and
natural essential oil.

5. A clathrate compound according to claim 4, wherein said hydroquinone compound is 2,5-di-tert-butylhydroquinone.

6. A clathrate compound according to claim 4 or claim 5, wherein said essential oil is one of cineole, hinoki oil, fragrant olive oil, jasmine oil, lemon oil, quassia oil, cinnamon oil, menthol, rosemary oil, palmarosa oil, lavender oil, spearmint oil and

7. A method of producing a clathrate compound comprising:
mixing a hydroquinone compound represented by general formula (I)

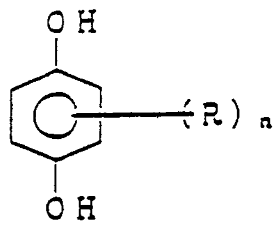

(I)

where R is a branched alkyl group having 3 to 7 carbon atoms, and n is 1 or 2, with a water-soluble microbicide or natural esential oil; and
obtaining a solid reaction product.

8. A method of producing a clathrate compound comprising:
mixing a solution or dispersion of a hydroquinone compound represented by general formula (I)

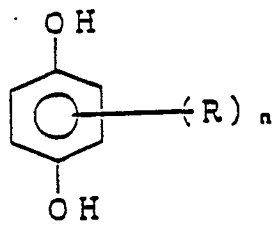

(I)

where R is a branched alkyl group having 3 to 7 carbon atoms, and n is 1 or 2, with a solution containing a water-soluble microbicide, or with natural essential oil, whereby a solid reaction product is obtained; and
separating said product from a liquid phase.